(19) Europäisches Patentamt
European Patent Office
Office européen des brevets

(11) Veröffentlichungsnummer: **0 339 264 B1**

(12) **EUROPÄISCHE PATENTSCHRIFT**

(45) Veröffentlichungstag der Patentschrift: **03.08.94**

(51) Int. Cl.$^5$: **C07D 213/71**, C07B 45/06, //C07K3/08

(21) Anmeldenummer: **89105157.5**

(22) Anmeldetag: **22.03.89**

(54) **Verbindungen zur Einführung einer SH-Gruppe in Tyrosin.**

(30) Priorität: **24.03.88 DE 3809986**

(43) Veröffentlichungstag der Anmeldung:
**02.11.89 Patentblatt 89/44**

(45) Bekanntmachung des Hinweises auf die Patenterteilung:
**03.08.94 Patentblatt 94/31**

(84) Benannte Vertragsstaaten:
**AT BE CH DE ES FR GB GR IT LI LU NL SE**

(56) Entgegenhaltungen:
**EP-A- 0 196 007**
**EP-A- 0 240 200**
**GB-A- 1 597 756**

**JOURNAL OF IMMUNOLOGICAL METHODS, Band 80, 1985, Seiten 137-140; R.J.S. DUNCAN et al.: "A diazonium reagent which will introduce maleimide groups into proteins and peptides"**

(73) Patentinhaber: **BOEHRINGER MANNHEIM GMBH**

**D-68298 Mannheim(DE)**

(72) Erfinder: **Klein, Christian, Dr. rer. nat.**
**Blütenstrasse 16**
**D-8120 Weilheim(DE)**
Erfinder: **Kirch, Peter, Dr. rer. nat.**
**Weilheimer Strasse 26**
**D-8911 Rott(DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08**
**20**
**D-81635 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Verbindungen der allgemeinen Formel I

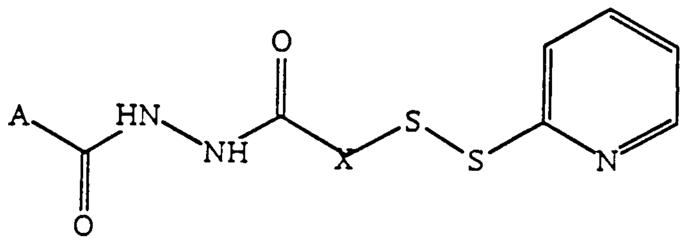

(I)

worin A einen aminsubstituierten Aromatenrest und X einen Kohlenwasserstoffrest mit 1 bis 10 Kohlenstoffatomen darstellt.

Zur Einführung von reaktiven Gruppen in Proteine sind eine Reihe von Reagenzien bekannt, die beispielsweise in Practice and Theory of Enzyme Immunoassays, P. Tijssen, Editors: R.H. Burdon, P.H. von Knippenberg, Elsevier-Verlag, 1985 beschrieben sind. Solche Reagenzien sind z.B. 2-Iminothiolan (Jue et al, Biochemistry 17, 5399-5405 (1978), N-Succinimidyl-3-(2-pyridyldithio)propionat (Carlsson et al, Biochem. J. 173, 723-737 (1978)), Acetylmercaptobernsteinsäureanhydrid (Klotz und Heiney, Arch. Biochem. Biophys. 96, 605-612 (1962)) oder S-Acetylmercaptoessigsäure-N-hydroxysuccinimidester (R. Julian et al, Anal. Biochem. 132, 68-73 (1983). Solche Verbindungen reagieren jeweils mit freien Aminogruppen des Proteins und können daher nur reaktive Gruppen in Proteine oder Peptide einführen, welche freie Aminogruppen wie ε-Aminogruppen von Lysin oder N-terminale Aminogruppen aufweisen. Es gibt jedoch Proteine, bei denen nur wenige oder keine reaktiven Aminogruppen zugänglich sind.

Ein Reagenz, mit dem eine SH-reaktive Gruppe an Tyrosin von Proteinen oder Peptiden gekoppelt werden kann, ist von Duncan et al., J. Immunol. Methods 80, 137-140 (1985) beschrieben worden. Dieses Reagenz muß jedoch in hohem Überschuß eingesetzt werden, so daß in beträchtlichem Umfang Nebenreaktionen ablaufen können.

Aufgabe der Erfindung war es daher, eine bifunktionelle Verbindung bereitzustellen, die eine Gruppe enthält, die an das Tyrosin gekoppelt werden kann und bei deren Bindung an Proteine oder Peptide Nebenreaktionen nur in geringem Umfang ablaufen.

Gelöst wurde diese Aufgabe erfindungsgemäß durch Verbindungen der allgemeinen Formel I

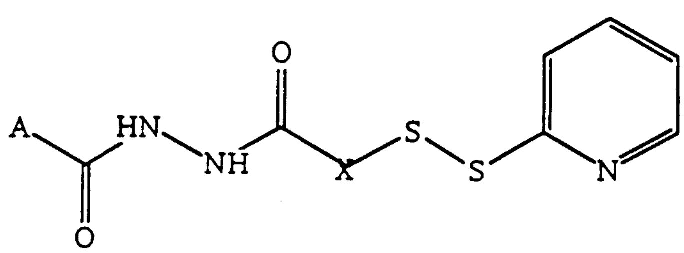

(I)

worin A einen aminsubstituierten Aromatenrest und X einen Kohlenwasserstoffrest mit 1 bis 10 Kohlenstoffatomen darstellt.

X ist bevorzugt ein Kohlenwasserstoffrest mit 1 bis 6 Kohlenstoffatomen und besonders bevorzugt ein Ethylidenrest.

Der aminsubstituierte aromatische Rest A kann beispielsweise nach an sich bekannten Methoden in ein Diazoniumsalz übergeführt werden, das wiederum leicht an den aromatischen Ring von Tyrosin oder einem Tyrosinderivat bindet, wonach durch Reduktion eine Thiopyridylgruppe abgespalten wird und eine SH-Gruppe erhalten wird.

Besonders bevorzugte erfindungsgemäße Verbindungen sind:

N-(4-Aminobenzoyl)-N'-(pyridyldithiopropionyl)-hydrazin

N-(4-Aminobenzoyl)-N'-(pyridyldithioacetyl)-hydrazin

N-(4-Aminobenzoyl)-N'-(pyridyldithiobutyryl)-hydrazin

N-(2-Aminobenzoyl)-N′-(pyridyldithiopropionyl)-hydrazin
N-(2-Aminobenzoyl)-N′-(pyridyldithioacetyl)-hydrazin
N-(2-Aminobenzoyl)-N′-(pyridyldithiobutyryl)-hydrazin.

Geeignet sind ebenfalls Verbindungen, bei denen der aminsubstituierte aromatische Rest A zusätzlich ein- oder mehrfach alkylsubstituiert ist. Die Aminsubstitution kann ein- oder mehrfach am aromatischen Rest A vorhanden sein.

Überraschenderweise wurde gefunden, daß von den erfindungsgemäßen Verbindungen bei der Kopplungsreaktion mit Proteinen oder Peptiden ca. 1/3 der Menge des eingesetzten Reagenzes eingebaut wird, so daß zur Reaktion nur ein ca. dreifacher Überschuß des Reagenzes eingesetzt werden muß.

Ein weiterer Gegenstand der Erfindung ist ein Verfahren zur Herstellung einer Verbindung der allgemeinen Formel I, wobei man einen Pyridyldithioalkylcarbonsäure-N-hydroxysuccinimidester mit 1 bis 10 Kohlenstoffatomen im Alkylteil des Carbonsäurerests mit dem Hydrazid einer am aromatischen Ring aminsubstituierten aromatischen Carbonsäure umsetzt.

Pyridyldithioalkylcarbonsäure-N-hydroxysuccinimidester erhält man aus den entsprechenden Pyridyldithioalkylcarbonsäuren durch Umsetzung mit N-Hydroxysuccinimid und einem Kondensationsmittel wie z.B. Dicyclohexylcarbodiimid, Diisopropylcarbodiimid oder Morpholinoethylisocyanid.

Ausgangsstoffe für die Pyridyldithioalkylcarbonsäuren sind die entsprechenden Mercaptoalkylcarbonsäuren. Sie werden durch Reaktion mit 2,2′-Pyridyldisulfid in die gewünschte Verbindung überführt.

Bevorzugt wird Pyridyldithiopropionsäure-N-hydroxysuccinimidester mit dem Hydrazid einer am aromatischen Ring aminsubstituierten aromatischen Carbonsäure umgesetzt.

Zur Herstellung der bevorzugten Verbindung N-(4-Aminobenzoyl)-N′-(pyridyldithiopropionyl)-hydrazin wird Pyridyldithiopropionsäure-N-hydroxysuccinimidester mit p-Aminobenzoesäurehydrazid umgesetzt.

Ein weiterer Gegenstand der Erfindung ist die Verwendung einer Verbindung der allgemeinen Formel I, bevorzugt von N-(4-Aminobenzoyl)-N′-(pyridyldithiopropionyl)-hydrazin, zur Einführung einer SH-Gruppe in ein Tyrosin oder Tyrosinderivat, insbesondere in ein in einem Peptid oder Protein gebundenes Tyrosin oder Tyrosinderivat. Hierbei wird die erfindungsgemäße Verbindung über die Aminsubstitution der Gruppe A an den aromatischen Ring des Tyrosins gebunden und durch Reduktion eine Thiopyridylgruppe abgespalten, wobei eine SH- Gruppe entsteht.

In einer bevorzugten Ausführungsform der Erfindung wird die Aminogruppe des Restes A vor der Umsetzung mit dem Tyrosin oder Tyrosinderivat nach an sich bekannten Methoden diazotiert, die diazotierte Verbindung sodann mit dem Tyrosin oder Tyrosinderivat umgesetzt und die Freisetzung der SH-Gruppe durch Einwirkung eines Reduktionsmittels bewirkt. Bevorzugt ist das Reduktionsmittel hierbei ein Thiol, wie beispielsweise Dithiotreitol.

Beispielhaft für geeignete Tyrosinderivate sind Thyroxin und Trijodthyronin. Beispielhaft für Proteine, in die eine SH-Gruppe erfindungsgemäß eingeführt werden kann sind RSA, IgG und HBsAg (Hepatitis-B-Oberflächen-Antigen).

Erfindungsgemäß wird es ermöglicht, SH-Gruppen an Tyrosinseitenketten einzuführen. Bei dem bevorzugten Einbau nach Diazotierung der erfindungsgemäßen Verbindung entsteht eine Diazogruppe, weshalb die Einbaurate durch einfache UV-Messung bestimmt werden kann. Die Freisetzung der SH-Gruppe erfolgt erfindungsgemäß selektiv erst durch Zugabe des Reduktionsmittels und kann ebenfalls durch UV-Messung verfolgt werden.

Erfindungsgemäß ist auch die Einführung einer SH-reaktiven Gruppe, nämlich der Pyridyldithiogruppe, also einer Gruppe, die mit SH-Gruppen reagieren kann, möglich. Es entfällt bei der Herstellung dann der Reduktionsschritt.

Die folgenden Beispiele erläutern die Erfindung weiter.

**Beispiel 1**

Synthese von N-(4-Aminobenzoyl)-N′-(pyridyldithiopropionyl)-hydrazin

242 mg p-Aminobenzoesäurehydrazid werden in 20 ml Dioxan/Wasser 3:1 aufgenommen. Dazu gibt man 500 mg Pyridyldithiopropionsäure-N-hydroxysuccinimidester. Man rührt 4 Stunden bei Raumtemperatur, dampft dann im Vakuum zur Trockne ein und kristallisiert aus Methanol um. Das Kristallisat wird abgesaugt, mit Chloroform gewaschen und im Vakuum über $CaCl_2$ getrocknet.
Ausbeute: 500 mg = 89 % der Theorie
$^1$H-NMR($[D]_6$-DMSO/$CD_3CO_2H$): $\delta$ = 2,66 (t, J = 6,6 Hz, 2 H); 3,09 (t, J = 6,6 Hz, 2 H); 6,6 (d, J = 7,2 Hz, 2 H); 7,22 (q, J = 4,5 Hz, 1 H); 7,63 (d, J = 7,2 Hz, 2 H); 7,81 (m, 2 H); 8,47 ppm (d, J = 4,5 Hz, 1 H).

**Beispiel 2**

Kopplung von N-(4-Aminobenzoyl)-N′-(pyridyldithiopropionyl)-hydrazin an Schaf-Immunoglobulin.

a) Herstellung der Reagenzlösung
5 mg N-(4-Aminobenzoyl)-N′-(pyridyldithiopropionyl)hydrazin (14,4 µmol) werden bei 0 °C in 0,2 ml 5 N HCl gelöst. Dazu gibt man 10 µl einer wäßrigen $NaNO_2$-Lösung (220 mg/ml), rührt 10 Minuten bei 0 °C, gibt 10 µl 2 M Harnstoff zu und rührt nochmals 10 Minuten bei 0 °C. Die Reaktionslösung wird mit eiskaltem Wasser auf 2 µmol/ml verdünnt und sofort eingesetzt.
b) Kopplung an Schaf-Immunoglobulin
500 µl Schaf-Immunglobulin (c = 20 mg/ml) in 0,1 M Boratpuffer pH 9,2 werden mit 500 µl 0,8 M Boratpuffer pH 9,2 versetzt. Bei 0 °C gibt man abwechselnd in mindestens 5 Portionen zu: 500 µl 0,14 M NaOH und 50 µl der oben beschriebenen Reagenzlösung. Damit werden pro mol Immunglobulin 15 mol Reagenz eingesetzt.

Man inkubiert 20 Minuten bei 0 °C, danach 20 Minuten bei 25 °C und entsalzt über Sephadex G 25.

Zur Bestimmung der Menge M an gebundenem Reagenz wird die Extinktion (OD) bei 420 nm bestimmt und nach folgender Formel ermittelt:

$$M\ (\text{mmol/l}) = \frac{OD_{420}}{3000}$$

Nach Bestimmung der Proteinkonzentration erhält man eine Einbaurate von 4,8 : 1.
c) Freisetzung der SH-Gruppen mit Dithiotreitol
Die Proteinlösung wird mit 2 M Essigsäure auf pH 4,5 eingestellt und mit Dithiotreitol ad 20 mM versetzt. Man inkubiert 20 Minuten bei 25 °C. Durch Extinktionsmessung bei 343 nm (Absorption des freigesetzten Thiopyridon) bestimmt man die freigesetzten SH-Gruppen. Der Extinktionsanstieg ($OD_{343}$) geteilt durch 8,08 ergibt die Konzentration an SH-Gruppen in µmol/ml.Man erhält einen Beladungsgrad von 5 SH-Gruppen/Molekül IgG.

Analog lassen sich bei HBsAg (Hepatitis B-Oberflächenantigen) und HBeAg (Hepatitis B-e-Antigen) 0,2 bis 2 mol SH-Gruppen/Mol Protein einbauen, je nach eingesetztem Überschuß des Reagenz. In der Regel werden ein Drittel des angebotenen Reagenz eingebaut.

## Patentansprüche

**Patentansprüche für folgende Vertragsstaaten : AT, BE, CH, DE, FR, GB, GR, IT, LI, LU, NL, SE**

**1.** Verbindungen der allgemeinen Formel

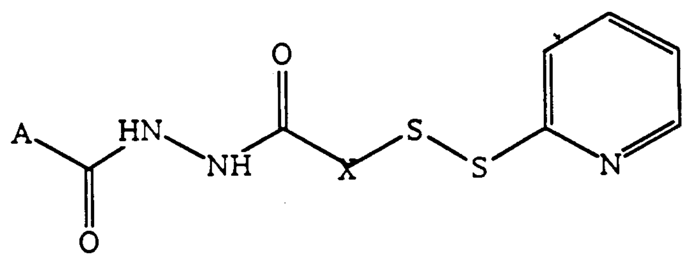

(I)

worin A einen aminsubstituierten Aromatenrest und X einen Kohlenwasserstoffrest mit 1 bis 10 Kohlenstoffatomen darstellt.

**2.** Verbindung nach Anspruch 1,
**gekennzeichnet durch**
die Formel N-(4-Aminobenzoyl)-N'-(pyridyldithiopropionyl)-hydrazin.

**3.** Verfahren zur Herstellung einer Verbindung nach Anspruch 1, **dadurch gekennzeichnet,** daß man einen Pyridyldithiocarbonsäure-N-hydroxysuccinimidester mit 1 bis 10 Kohlenstoffatomen im Alkylteil

des Carbonsäurerestes mit dem Hydrazid einer am aromatischen Ring aminsubstituierten aromatischen Carbonsäure umsetzt.

4. Verfahren zur Herstellung der Verbindung nach Anspruch 2, **dadurch gekennzeichnet,** daß man Pyridyldithiopropionsäure-N-hydroxysuccinimidester mit p-Aminobenzoesäurehydrazid umsetzt.

5. Verwendung einer Verbindung nach Anspruch 1 oder 2 zur Einführung einer SH-Gruppe in ein Tyrosin, insbesondere in ein in einem Peptid oder Protein gebundenes Tyrosin oder Tyrosinderivat.

6. Verwendung nach Anspruch 5, umfassend Diazotieren des aromatischen Amins A, Umsetzen der diazotierten Verbindung mit dem Tyrosin, seinem Derivat bzw. dem Tyrosin oder sein Derivat enthaltenden Peptid oder Protein und Freisetzen einer SH-Gruppe durch Einwirkung eines Reduktionsmittels.

**Patentansprüche für folgenden Vertragsstaat : ES**

1. Verfahren zur Herstellung einer Verbindung der allgemeinen Formel

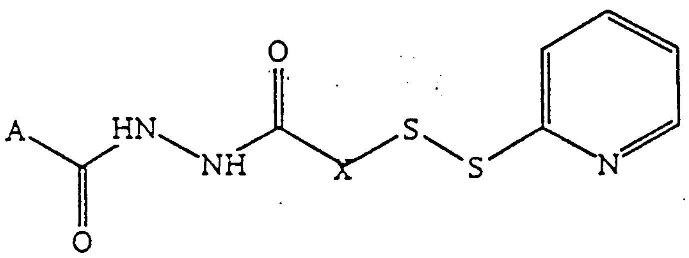

(I)

worin A einen aminsubstituierten Aromatenrest und X einen Kohlenwasserstoffrest mit 1 bis 10 Kohlenstoffatomen darstellt,
**dadurch gekennzeichnet**,
daß man einen pyridyldithiocarbonsäure-N-hydroxysuccinimidester mit 1 bis 10 Kohlenstoffatomen im Alkylteil des Carbonsäurerestes mit dem Hydrazid einer am aromatischen Ring aminsubstituierten aromatischen Carbonsäure umsetzt.

2. Verfahren nach Anspruch 1,
**dadurch gekennzeichnet,**
daß man Pridyldithiopropionsäure-N-hydroxysuccinimidester mit p-Aminobenzoesäurehydrazid umsetzt unter Bildung von N-(4-Aminobenzoyl)-N'-(pyridyldithiopropionyl)-hydrazin.

3. Verfahren zur Einführung einer SH-Gruppe in ein Tyrosin, insbesondere in ein in einem Peptid oder Protein gebundenes Tyrosin oder Tyrosinderivat,
**dadurch gekennzeichnet,**
daß man eine nach Anspruch 1 oder 2 hergestellte Verbindung verwendet.

4. Verfahren nach Anspruch 3, umfassend Diazotieren des aromatischen Amins A, Umsetzen der diazotierten Verbindung mit dem Tyrosin, seinem Derivat bzw. dem Tyrosin oder sein Derivat enthaltenden Peptid oder Protein und Freisetzen einer SH-Gruppe durch Einwirkung eines Reduktionsmittels.

## Claims

### Claims for the following Contracting States : AT, BE, CH, DE, FR, GB, GR, IT, LI, LU, NL, SE

1. Compounds of the general formula:-

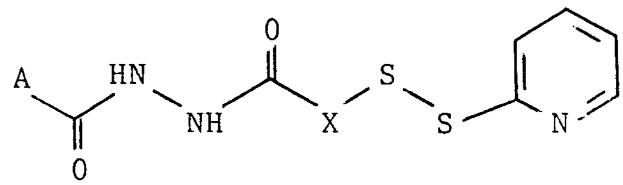

(I)

wherein A represents an amine-substituted aromatic radical and X a hydrocarbon radical with 1 to 10 carbon atoms.

2. Compound according to claim 1, characterised by the formula N-(4-aminobenzoyl)-N'-(pyridyldithiopropionyl)-hydrazine.

3. Process for the preparation of a compound according to claim 1, characterised in that one reacts a pyridyldithiocarboxylic acid N-hydroxysuccinimide ester with 1 to 10 carbon atoms in the alkyl part of the carboxylic acid residue with the hydrazide of an aromatic carboxylic acid amino-substituted on the aromatic ring.

4. Process for the preparation of the compound according to claim 2, characterised in that one reacts pyridyldithiopropionic acid N-hydroxysuccinimide ester with p-aminobenzoic acid hydrazide.

5. Use of a compound according to claim 1 or 2 for the introduction of an SH group into a tyrosine, especially into a tyrosine or tyrosine derivative bound in a protein or peptide.

6. Use according to claim 5, including diazotisation of the aromatic amine A, reaction of the diazotised compound with the tyrosine, its derivative or peptide or protein containing the tyrosine or its derivative thereof and liberation of an SH group by action of a reducing agent.

### Claims for the following Contracting State : ES

1. Process for the preparation of a compound of the general formula

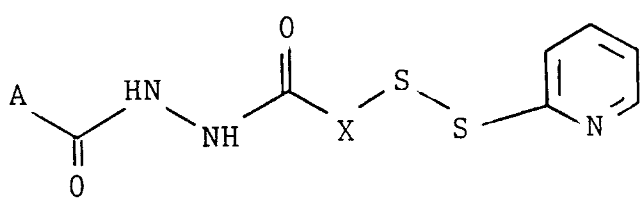

(I)

wherein A represents an amine-substituted aromatic radical and X a hydrocarbon radical with 1 to 10 carbon atoms, characterised in that one reacts a pyridyldithiocarboxylic acid N-hydroxysuccinimide ester with 1 to 10 carbon atoms in the alkyl part of the carboxylic acid residue with the hydrazide of an aromatic carboxylic acid amine-substituted on the aromatic ring.

2. Process according to claim 1, characterised in that one reacts pyridyldithiopropionic acid N-hydroxysuccinimide ester with p-aminobenzoic acid hydrazide with formation of N-(4-aminobenzoyl)-N'-(pyridyldithiopropionyl)-hydrazine.

3. Process for the introduction of an SH group into a tyrosine, especially into a tyrosine or tyrosine derivative bound in a peptide or protein, characterised in that one uses a compound prepared

according to claim 1 or 2.

4. Process according to claim 3, including diazotisation of the aromatic amine, reaction of the diazotised compound with the tyrosine, its derivative or the peptide or protein containing tyrosine or its derivative and liberation of an SH group by action of a reducing agent.

**Revendications**

**Revendications pour les Etats contractants suivants : AT, BE, CH, DE, FR, GB, GR, IT, LI, LU, NL, SE**

1. Composés de formule générale

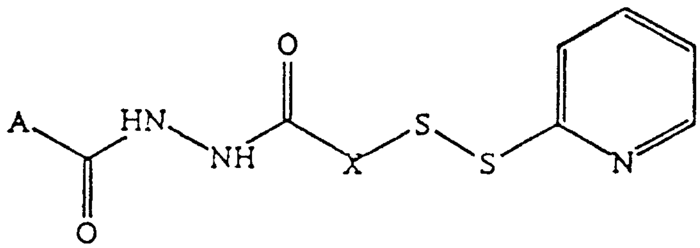

(I)

dans laquelle A représente un reste aromatique aminosubstitué et X représente un reste hydrocarboné de 1 à 10 atomes de carbone.

2. Composé selon la revendication 1, caractérisé par la formule N-(4-aminobenzoyl)-N'-(pyridyldithiopropionyl)hydrazine.

3. Procédé de préparation d'un composé selon la revendication 1, caractérisé en ce que l'on fait réagir un pyridyldithioalkylcarboxylate de N-hydroxysuccinimide ayant 1 à 10 atomes de carbone dans la partie alkyle du reste acide carboxylique avec l'hydrazide d'un acide carboxylique aromatique aminosubstitué sur le cycle aromatique.

4. Procédé de préparation du composé selon la revendication 2, caractérisé en ce que l'on fait réagir le pyridyldithiopropionate de N-hydroxysuccinimide avec l'hydrazide de l'acide p-aminobenzoïque.

5. Utilisation d'un composé selon la revendication 1 ou 2 pour l'introduction d'un groupe SH dans une tyrosine, en particulier dans une tyrosine liée dans un peptide ou une protéine ou dans un dérivé de tyrosine lié dans un peptide ou une protéine.

6. Utilisation selon la revendication 5, comprenant la diazotation de l'amine aromatique A, la réaction du composé diazoté avec la tyrosine, son dérivé ou le peptide ou la protéine contenant la tyrosine ou son dérivé, et la libération d'un groupe SH par action d'un réducteur.

**Revendications pour l'Etat contractant suivant : ES**

1. Procédé de préparation d'un composé de formule générale

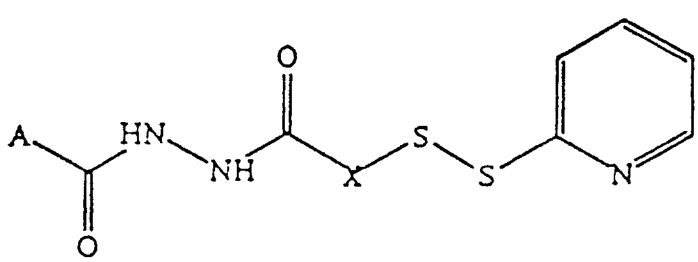

(I)

dans laquelle A représente un reste aromatique aminosubstitué et X représente un reste hydrocarboné de 1 à 10 atomes de carbone, caractérisé en ce que l'on fait réagir un pyridyldithioalkylcarboxylate de N-hydroxysuccinimide ayant 1 à 10 atomes de carbone dans la partie alkyle du reste acide carboxylique avec l'hydrazide d'un acide carboxylique aromatique aminosubstitué sur le cycle aromatique.

**2.** Procédé selon la revendication 1, caractérisé en ce que l'on fait réagir le pyridyldithiopropionate de N-hydroxysuccinimide avec l'hydrazide de l'acide p-aminobenzoïque avec formation de N-(4-aminobenzoyl)-N'-(pyridyldithiopropionyl)hydrazine.

**3.** Procédé pour introduire un groupe SH dans une tyrosine, en particulier dans une tyrosine liée dans un peptide ou une protéine ou dans un dérivé de tyrosine lié dans un peptide ou une protéine, caractérisé en ce que l'on utilise un composé préparé selon la revendication 1 ou 2.

**4.** Procédé selon la revendication 3, comprenant la diazotation de l'amine aromatique A, la réaction du composé diazoté avec la tyrosine, son dérivé ou le peptide ou la protéine contenant la tyrosine ou son dérivé, et la libération d'un groupe SH par action d'un réducteur.